(19) Europäisches Patentamt
European Patent Office
Office européen des brevets

(11) Publication number: **0 059 646**
**A2**

# (12) EUROPEAN PATENT APPLICATION

(21) Application number: 82301081.4

(22) Date of filing: 03.03.82

(51) Int. Cl.³: C 08 G 61/12
C 07 D 333/08, C 07 D 213/06
C 07 D 307/36, C 07 D 207/32

(30) Priority: 03.03.81 JP 29378/81
03.03.81 JP 29379/81
11.03.81 JP 34005/81
11.03.81 JP 34006/81
17.03.81 JP 37311/81
30.07.81 JP 118463/81
30.11.81 JP 190668/81
04.12.81 JP 194452/81

(43) Date of publication of application:
08.09.82 Bulletin 82/36

(84) Designated Contracting States:
DE FR GB NL

(71) Applicant: JAPAN SYNTHETIC RUBBER CO., LTD.
11-24, Tsukiji-2-chome Chuo-ku
Tokyo(JP)

(72) Inventor: Nozue, Ikuo
29, Aobadai-2-chome Midori-ku
Yokohama(JP)

(72) Inventor: Yumoto, Yoshiji
29 Aobadai 2-chome Midori-ku
Yokohama(JP)

(72) Inventor: Matsumura, Yoshio
14-30, Tsukimino 8-chome
Yamato-shi(JP)

(74) Representative: Tubby, David George et al,
MARKS & CLERK 57-60 Lincoln's Inn Fields
London WC2A 3LS(GB)

## (54) Conjugated polymers and processes for preparing and modifying them.

(57) A conjugated polymer having a recurring unit of formula (I) or (II):

$$-Ar-C\equiv C-Ar'-C\equiv C- \qquad (I)$$

$$-C\equiv C-Ar-C\equiv C- \qquad (II)$$

(in which Ar and Ar' are the same or different and each represents a heteroaromatic ring) has good heat stability and mouldability. The polymer having a recurring unit of formula (I) may be modified by reaction with an amine or with hydrogen sulphide and both may be modified by doping with an electron-donative compound or an electron-attractive compound, to prepare organic semiconductors or organic conductors having various electrical conductivities.

EP 0 059 646 A2

CONJUGATED POLYMERS AND PROCESSES FOR PREPARING AND MODIFYING THEM

The present invention relates to novel conjugated polymers containing ethynylene groups and heteroaromatic rings, to processes for producing these polymers and to processes for modifying them.

A number of polymers containing conjugated ethynylene groups and aromatic rings are known, specifically the meta- and para- forms of poly(diethynylbenzene) are disclosed in J. Polymer Sci., A-1, 7, 1625 (1969) and polymers of the type having a repeating unit of formula:

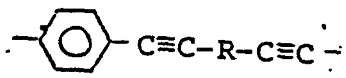

(wherein R represents, for example, a thiophene or pyridine ring) have very recently been reported in Polymer Preprints, Japan, 30, No. 1, 160 (1981).

Of the poly(diethynylbenzene) polymers, the para- form decomposes thermally at 100°C. The heat resistance of poly(meta-diethynylbenzene) can be higher but depends upon the purity of the meta-diethynylbenzene used as the starting material. However, it is very difficult to synthesize very pure meta-diethynylbenzene and thus, most commonly, poly(meta-diethynylbenzene) decomposes thermally at 200°C as disclosed in J. Org. Chem., 25, 637 (1960).

One of the starting materials used in the preparation of polymers having a repeating unit of formula:

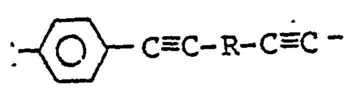

is para-diethynylbenzene and this is also difficult to synthesize in high purity. Even though these polymers have superior heat resistance to the poly(diethynylbenzene)s, they have inadequate mouldability and processability because of their insolubility in organic solvents.

We have now discovered a class of conjugated polymers containing ethynylene groups and heteroaromatic groups, which can have excellent heat stability and good mouldability.

Accordingly, the present invention consists in a conjugated polymer having a recurring unit of formula (I) or of formula (II):

$$-C\equiv C-Ar-C\equiv C- \qquad (I)$$

$$-Ar-C\equiv C-Ar'-C\equiv C- \qquad (II)$$

(in which Ar and Ar' are the same or different and each represents a heteroaromatic ring).

The invention also provides a process for preparing a conjugated polymer having a recurring unit of the aforementioned formula (I) in which a diethynyl-heteroaromatic compound of formula (III):

$$HC\equiv C-Ar-C\equiv CH \qquad (III)$$

(in which Ar is as defined above) is subjected to an oxidative coupling reaction with oxygen in the presence of a catalyst comprising a copper compound and an amine compound.

The invention also provides a process for preparing a conjugated polymer having a recurring unit of the aforementioned formula (II) in which a diethynyl-heteroaromatic compound of the aforementioned formula (III) is reacted with a dihalo-heteroaromatic compound of formula (IV):

$$X-Ar'-X, \qquad (IV)$$

(in which Ar' is as defined above and X represents a halogen atom) in the presence of an amine compound and of a catalyst comprising a palladium compound and a copper compound.

There is still further provided a process for producing a conjugated polymer having a recurring unit of the aforementioned formula (II) in which a dihalo-heteroaromatic compound of the aforementioned formula (IV) is reacted with a di-Grignard reagent of formula (V):

$$X'MgC{\equiv}C-Ar-C{\equiv}CMgX' \qquad (V)$$

[in which Ar is as defined above and X' represents a halogen atom, which may be the same as or different from that represented by X in the compound of formula (IV)] in the presence of a palladium- and phosphorus-containing catalyst.

In the aforementioned formulae, the heteroaromatic rings represented by Ar and Ar' are heterocyclic rings having an aromatic character, including, for example, the thiophene, pyridine,

furan and pyrrole rings, of which the thiophene and pyridine rings are preferred.

The processes for preparing the polymers of the present invention will now be described in more detail.

PROCESS A

In this process, a conjugated polymer having a recurring unit of formula (I):

$$-C{\equiv}C-Ar-C{\equiv}C- \quad (I)$$

is prepared by subjecting a diethynyl-heteroaromatic compound of formula (III):

$$HC{\equiv}C-Ar-C{\equiv}CH \quad (III)$$

to oxidative coupling with oxygen in the presence of a catalyst comprising a copper compound and an amine compound.

Examples of diethynyl-heteroaromatic compounds of formula (III) which may be used in this process include the diethynylthiophenes, diethynylpyridines, diethynylfurans and diethynylpyrroles, of which we prefer to use the diethynylthiophenes and diethnylpyridines, since these are relatively easy to synthesize.

Amine compounds, which are one of the components of the catalyst in this process, may be primary amines (such as hexylamine, aniline or benzylamine), secondary amines (such as dimethylamine, diethylamine, methylethylamine, N-methylaniline or piperidine) or tertiary amines (such as trimethylamine, triethylamine, N,N-dimethylaniline, benzyldimethylamine or N,N,N',N'-tetramethylethylenediamine) of which we prefer to use amines having a boiling point within the range from 50 to 150°C (such as diethylamine, triethylamine or N,N,N',N'-tetramethylethylenediamine) as this facilitates subsequent treatment of the reaction mixture. The amount of amine is preferably at least 0.005 mole, more preferably from 0.01 to 0.1 mole, per mole of said diethynyl-heteroaromatic compound of formula (III).

The copper compound, which is the other component of the catalyst used in this process, is preferably a monovalent copper compound, such as cuprous chloride, cuprous iodide, cuprous bromide, cuprous oxide or cuprous cyanide, of which cuprous bromide, cuprous iodide and cuprous chloride are preferred. The amount of this copper compound is preferably at least 0.005 mole, more preferably from 0.01 to 0.1 mole, per mole of the diethynyl-heteroaromatic compound.

In carrying out this process, the diethynyl-heteroaromatic compound and the catalysts, that is the copper and amine compounds,

are first dissolved in a solvent, the nature of which is not critical, provided that it has no adverse effect upon the reaction. Suitable solvents include hydrocarbons, ethers, amines, alcohols, ketones, esters and nitro compounds. However, since the molecular weight of the conjugated polymer formed is often dependent upon the solubility of the polymer in the solvent employed, the use of nitrogen-containing compounds (such as nitrobenzene, pyridine, N-methylpyrrolidone or hexamethylphosphoric triamide) is preferred when producing conjugated polymers having a high molecular weight. The oxygen necessary for the oxidative coupling reaction is preferably then blown into the solution. The reaction temperature is preferably within the range from 40°C to 150°C and the oxygen is preferably blown through the solution for a period of from 2 to 24 hours.

After cooling the reaction mixture, the solvent is then preferably removed by evaporation under reduced pressure and the copper compound is then removed, for example by treatment with an alcoholic solution of an acid (for example a methanolic solution of hydrochloric acid). The resulting solution is then suitably filtered to give a powder, which is then washed with a basic solution (for example a methanolic solution of an alkali metal hydroxide) to give the conjugated polymer of the present invention, normally as a dark-brown powder.

PROCESS B

In this process, a conjugated polymer having a recurring unit of formula (II):

$$-Ar-C\equiv C-Ar'-C\equiv C- \qquad (II)$$

is prepared by reacting a diethynyl-heteroaromatic compound of formula (III):

$$HC\equiv C-Ar-C\equiv CH \qquad (III)$$

with a dihalo-heteroaromatic compound of formula (IV):

$$X-Ar'-X \qquad (IV)$$

in the presence of an amine compound and of a catalyst comprising a palladium compound and a copper compound.

Suitable diethynyl-heteroaromatic compounds, amine compounds and copper compounds for use in this process are as described in connection with Process A.

The amount of amine used is preferably at least 5 moles per mole of the diethynyl-heteroaromatic compound (III) and the amount of copper compound is preferably from 0.001 to 0.01 mole per mole of the diethynyl-heteroaromatic compound.

Suitable palladium compounds for use in this process include $Pd(PR^6_3)_4$, $Pd(PR^6_3)_2X^2_2$, $Pd(AsR^6_3)_2X^2_2$, $Pd(NR^6_3)_2X^2_2$, and $Pd(OCOR^6)_2$ (in which $R^6$ represents an alkyl, phenyl or aralkyl group and $X^2$ represents ~~a~~ halogen atom, which may be the same as or different from that represented by X and $X^1$), of which $Pd(PPh_3)_4$, $Pd(PPh_3)Cl_2$ and $Pd(OCOCH_3)$ (Ph represents a phenyl group) are particularly preferred. The amount of palladium compound is preferably from 0.001 to 0.1 mole per mole of diethynyl-heteroaromatic compound (III).

In a preferred embodiment of this process of the invention, approximately equimolar amounts of the diethynyl-heteroaromatic compound (III) and the dihalo-heteroaromatic compound (IV), together with at least 5 moles of the amine (per mole of the diethynyl-heteroaromatic compound), are dissolved in a solvent, suitable solvents being those described in Process A. Then the catalysts, that is the palladium compound and the copper compound, are added to the solution, which is then stirred.

The reaction temperature is preferably from 50°C to 120°C and the time required for the reaction is generally from 1 to 5 hours.

At the end of the reaction time, the reaction mixture is preferably cooled, the solution is concentrated by evaporation

under reduced pressure and the concentrate is washed with an aqueous acid (e.g. aqueous hydrochloric acid) to remove the ammonium halide and the residual catalysts, after which it is washed with a basic solution (such as a methanolic solution of an alkali metal hydroxide). The result is the conjugated polymer of the present invention in the form of a dark-brown powder.

The dihalo-heteroaromatic compound of formula (III) employed in this process will, of course, depend upon the group Ar' which it is desired to incorporate in the final product, but examples of these compounds include the 2,3-, 2,4-, 3,4- and 2,5- dihalothiophenes and the 2,3-, 2,4-, 2,5-, 2,6-, 3,4- and 3,5- dihalopyridines.

PROCESS C

A conjugated polymer having a recurring unit of formula (II):

$$-Ar-C{\equiv}C-Ar'-C{\equiv}C- \qquad (II)$$

is prepared by reacting a dihalo-heteroaromatic compound of formula (IV):

$$X-Ar'-X \qquad (IV)$$

with a di-Grignard reagent of formula (V):

$$X'MgC{\equiv}C{-}Ar{-}C{\equiv}CMgX' \quad (V)$$

in the presence of a palladium- and phosphorus- containing catalyst.

Suitable dihalo-heteroaromatic compounds of formula (IV) are those suggested above in relation to Process B. Examples of palladium compounds and preferred palladium compounds for use in Process C are also the same as in Process B and the preferred amount of palladium compound used as catalyst is from 0.001 to 0.1 mole per mole of the di-Grignard reagent (V). Where the palladium compound contains phosphorus, the catalyst need not contain any additional phosphorus compound; if, however, the palladium compound is one of those which does not contain phosphorus, then an additional phosphorus compound is necessary as part of the catalyst in Process C.

Suitable phosphorus compounds which may be employed include triphenylphosphine, trimethylphosphine, triethylphosphine, tripropylphosphine, tributylphosphine, tripentylphosphine, dimethylphenylphosphine, methyldiphenylphosphine and tris(dimethylamino)phosphine. Where a phosphorus compound is used, the amount is preferably from 2 to 5 moles per mole of the palladium compound in the catalyst.

In carrying out Process C, we prefer that the di-Grignard reagent (V) and an approximately equimolar amount of the dihalo-heteroaromatic compound (IV) are first dissolved in a solvent, such as an ether. The catalysts, that is the palladium compound and, if necessary, the phosphorus compound, are then added to the solution and the resulting mixture is stirred.

The reaction temperature is preferably from 40°C to 140°C and the time required for the reaction is normally from 2 to 5 hours.

After completion of the reaction, the resulting solid is washed with an aqueous acid (e.g. aqueous hydrochloric acid) to remove residual catalysts and then with a basic solution (such as a methanolic solution of an alkali metal hydroxide). The product is the conjugated polymer of the invention in the form of a dark-brown powder.

The diethynyl-heteroaromatic compounds of formula (III), which are one of the starting materials used in Processes A and B may, for example, be prepared by any of the following Processes D, E and F.

Process D

Diethynyl-heteroaromatic compounds can be prepared by

reacting a dihalo-heteroaromatic compound with an ethynylsilyl compound of formula (VI):

$$R^1R^2R^3SiC{\equiv}CH \qquad (VI)$$

(in which $R^1$, $R^2$ and $R^3$ are the same or different and each represents an alkyl, aryl or alkenyl group) in the presence of a dehalogenating agent and a catalyst comprising a palladium compound and a copper compound, followed by de-silylating the reaction product with a metal halide.

Suitable dihalo-heteroaromatic compounds for use in this process include dihalothiophenes, dihalopyridines, dihalopyrroles and dihalofurans, of which the dihalothiophenes and dihalopyridines are preferred because of the ease with which they react, the dihalothiophenes being particularly preferred. The halogen atoms in these dihalo-heteroaromatic compounds are preferably chlorine or bromine atoms.

Examples of the groups represented by $R^1$, $R^2$ and $R^3$ in the ethynylsilyl compound of formula (VI) include such alkyl groups as the methyl, ethyl and propyl groups, such aryl groups as the phenyl group and such alkenyl groups as the vinyl and isopropenyl groups, of which alkyl and aryl groups are preferred

because of the ease with which they react, the methyl group being particularly preferred.

The amount of ethynylsilyl compound (VI) is preferably from 2 to 2.5 moles per mole of the dihalo-heteroaromatic compound.

Suitable palladium compounds for use as the catalyst in this process have been illustrated in relation to Process B, preferred compounds for use in Process D being those of formula $Pd(PPh_3)_2Br_2$ and $Pd(PPh_3)_2Cl_2$ ("Ph" represents a phenyl group). These palladium compounds are preferably used in an amount of from 0.01 to 0.1 mole per mole of the dihalo-heteroaromatic compound.

Suitable copper compounds for use as the other component of the catalyst in this process have been illustrated in relation to Process A. These copper compounds are preferably used in an amount of from 0.005 to 0.1 mole per mole of said ethynylsilyl compound (VI).

The dehalogenating agent used in this process is most preferably an amine. Any amine may be used, provided that it is capable of forming an ammonium halide and thus suitable amines include primary amines (such as hexylamine, aniline and benzylamine), secondary amines (such as dimethylamine, diethylamine, methylethylamine, N-methylaniline and piperidine) and tertiary amines (such as

trimethylamine, triethylamine, N,N-dimethylaniline, benzyldimethylamine and N,N,N',N'-tetramethylethylenediamine), of which we prefer to use amines having a boiling point of from 50°C to 150°C (such as diethylamine, triethylamine or N,N,N',N'-tetramethylethylenediamine) as this facilitates subsequent treatment of the reaction mixture. The amount of dehalogenating agent used is preferably at least 5 moles, and more preferably from 5 to 1000 moles, per mole of the dihalo-heteroaromatic compound.

Although this amine can also serve as the reaction solvent, it is possible to use one or more other solvents together with the amine. Suitable such additional solvents include hydrocarbons (e.g. hexane or benzene), halogenated hydrocarbons (e.g. methylene chloride or chloroform) and ethers (e.g. diethyl ether or tetrahydrofuran).

The preferred de-silylating agents for use in this process are metal halides, particularly alkali metal halides, such as potassium fluoride, potassium bromide, potassium iodide, potassium chloride, sodium iodide, sodium fluoride, sodium chloride, sodium bromide, lithium fluoride, lithium iodide, lithium chloride or lithium bromide, of which potassium fluoride and sodium fluoride are preferred. The amount of de-silylating agent used is preferably from 0.01 to 1 mole per mole of the intermediate compound of formula (VII):

$$R^1R^2R^3SiC{\equiv}C\text{-}Ar\text{-}C{\equiv}CSiR^1R^2R^3 \qquad (VII)$$

which is formed by the reaction of the dihalo-heteroaromatic compound and the ethynylsilyl compound (VI).

In carrying out this process, we prefer that the dihalo-heteroaromatic compound and the ethynylsilyl compound (VI) should first be dissolved in a solvent containing or consisting of an amine. After adding to the solution a palladium compound and a copper compound as catalysts, the resulting mixture is heated, with stirring. The mixture is preferably heated to a temperature of from 20°C to 100°C and the time required for the reaction will generally be from 1 to 8 hours. Although the nature of the atmosphere under which the reaction is carried out is not critical, we prefer to carry out the reaction in an atmosphere of an inert gas, such as nitrogen. This reaction produces an ammonium halide and the aforementioned intermediate compound of formula (VII). Upon completion of this reaction, the insoluble by-product, i.e. the ammonium halide, is removed by filtration and the low boiling point substances in the filtrate are removed by distillation under reduced pressure. The residue is then extracted with a solvent for the intermediate (VII), for example benzene.

The extracted solution is then preferably treated with

activated carbon in order to remove any small quantity of impurities from the extract. Although any amount of activated carbon may be used, we prefer to use from 0.2 to 2 parts by weight of activated carbon per part by weight of dihalo-heteroaromatic compound, since too small an amount removes inusufficient of the impurities whilst too large an amount decreases the yield of intermediate (VII). After the treatment with activated carbon, the carbon is removed by filtration and the filtrate is evaporated to dryness, whereupon crystals of the intermediate (VII) are deposited.

Subsequently, these crystals are reacted with a metal halide (as previously exemplified) in an organic solvent, after which the solvent is removed. The organic solvent should be one in which the metal halide and the intermediate (VII) can dissolve, for example an ether, amine, ketone, aldehyde, sulphoxide, alcohol or ester. The reaction is preferably carried out at a temperature of from 40°C to 100°C and the reaction time is generally at least 1 hour. Although the atmosphere under which the reaction is carried out is not critical, we prefer to carry out the reaction under an atmosphere of an inert gas, such as nitrogen. The resulting reaction mixture is then extracted with a suitable solvent (such as diethyl ether) to remove the metal halide, after which the extraction solvent is removed by distillation, to give the diethynyl-heteroaromatic compound as an oil.

Process E

Diethynyl-heteroaromatic compounds can also be produced by reacting a dihalo-heteroaromatic compound with a propargyl alcohol derivative in the presence of a dehalogenating agent and of a catalyst comprising a palladium compound and a copper compound, followed by the deketonating the reaction product with a suitable deketonating agent.

Suitable dihalo-heteroaromatic compounds, palladium compounds, copper compounds, dehalogenating agents and reaction solvents for use in this process are all as illustrated in relation to Process D.

The propargyl alcohol derivative is suitably a compound of formula (VIII):

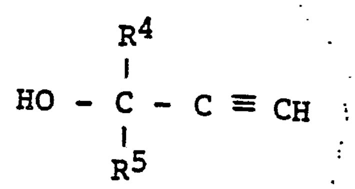

(VIII)

in which $R^4$ and $R^5$ are the same or different and each represents an alkyl group (e.g. methyl, ethyl or propyl), an aryl group (e.g. phenyl) or an alkenyl group (e.g. vinyl or isopropenyl);

we prefer that $R^4$ and $R^5$ should both represent methyl groups, since such a compound is commercially available and since this facilitates the subsequent deketonating reaction. This propargyl alcohol derivative is preferably used in an amount of from 2.0 to 3.0 moles per mole of the dihalo-heteroaromatic compound.

The deketonating agent is preferably a basic substance, for example an alkali metal (e.g. lithium or sodium), an alkali metal hydroxide (e.g. sodium hydroxide or potassium hydroxide), an alkali metal alkoxide (e.g. sodium methoxide or sodium ethoxide) or an alkyl-alkali metal (e.g. butyllithium), of which sodium hydroxide and potassium hydroxide are preferred. The amount of deketonating agent is preferably from 0.1 to 1 mole per mole of the intermediate compound of formula (IX):

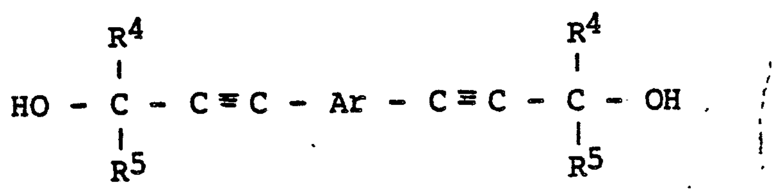 (IX)

(in which $R^4$ and $R^5$ are as defined above), which is prepared by the reaction between the dihalo-heteroaromatic compound and the propargyl alcohol derivative (VIII).

In a preferred way of carrying out this process, the dihalo-heteroaromatic compound and the propargyl alcohol derivative, e.g. the compound of formula (VIII), are dissolved in a solvent

containing a dehalogenating agent such as an amine. The palladium and copper compounds are then added, as catalysts, and the resulting mixture is allowed to react, with stirring. The reaction temperature is preferably from 15 to 70°C and the time required for the reaction will generally be from 10 to 20 hours. Although the atmosphere under which the reaction is carried out is not critical, we prefer to employ an inert atmosphere, for example one of nitrogen.

This reaction forms an ammonium halide and the aforementioned intermediate, e.g. the compound of formula (IX). After completion of the reaction, the ammonium halide is removed by washing the reaction mixture with water, and then the mixture is extracted with an ether. The low boiling point substances are then removed by distillation under reduced pressure, after which the residue is purified by distillation under reduced pressure.

The resulting intermediate compound (IX) is then subjected to deketonation by reacting it with a deketonating agent (particularly an alkali metal hydroxide) in an organic solvent, after which the resulting insoluble substances are removed by filtration and the filtrate is concentrated. The organic solvent may be any substance in which the intermediate (IX) can dissolve and suitable compounds include hydrocarbons, ethers, amines, aldehydes, sulphoxides, alcohols and esters. The temperature at which the

deketonation reaction is carried out is preferably from 50°C to 150°C and the time required for the reaction will generally be from 1 to 5 hours. Although the atmosphere under which the reaction is carried out is not critical, we prefer to employ an inert atmosphere, for example an atmosphere of nitrogen gas. On purifying the reaction product, there is obtained a diethynylheteroaromatic compound of formula (III).

Process F

2,6-Diethynylpyridine can also be produced by hitherto known processes, for example, by reacting a 2,6-dihalopyridine and trimethylsilylacetylene in an amine in the presence of a catalytic amount of bis(triphenylphosphine)-dichloropalladium and cuprous iodide, and then treating the resulting 2,6-bis(trimethylsilylethynyl)-pyridine with a dilute aqueous solution of potassium hydroxide in methanol at room temperature ["Synthesis", p. 627 (1980)].

When the conjugated polymer of the invention is prepared by Process A, the terminal atoms on the polymer chain are usually hydrogen atoms. When the polymer is produced by Processes B and C, the terminal atoms on the polymer chain are usually hydrogen

or halogen atoms. The degree of polymerisation of these polymers is not critical to the present invention; however, they are usually obtained as a product having a degree of polymerisation within the range from 10 to 1000; although products outside this range are also obtainable. Products having a degree of polymerisation within the range from 10 to 400 are particularly easy to produce.

It is also possible to modify those conjugated polymers of the present invention which have a repeating unit represented by the aforementioned formula (I), by employing either of the following Proces G and H.

Process G

In this process, a conjugated polymer having a recurring unit of formula (I):

$$-C\equiv C-Ar-C\equiv C- \qquad (I)$$

is reacted with an amine of formula (X):

$$NH_2R^7 \qquad (X)$$

($R^7$ represents a hydrogen atom or an alkyl, aryl or aralkyl group) in the presence of a copper compound, to convert all or some

of the diethynylene structures (which have the formula $-C\equiv C-C\equiv C-$) into a structure of formula (XI):

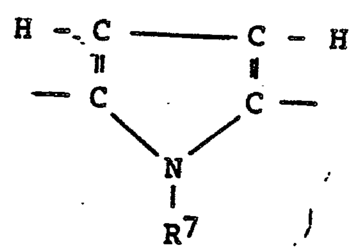

(XI)

In the amine of formula (X) used in this process, $R^7$ represents a hydrogen atom or an alkyl group (e.g. methyl, ethyl, propyl or hexyl), an aryl group (e.g. phenyl or tolyl) or an aralkyl group (e.g. benzyl or diphenylmethyl). Examples of such compounds include the alkylamines (such as methylamine, propylamine or hexylamine), the aromatic amines (such as aniline or p-toluidine) and the aralkylamines (such as benzylamine), as well as ammonia itself. In order to ensure that all of the diethynylene structures are reacted with the amine, we prefer to use from 5 to 100 equivalents of amine per diethynylene structure in the conjugated polymer, in view of the reactivity of these amines. If the amount of amine is less than 1 equivalent per diethynylene structure, then only some of these structures will, of course, react with the amine and be converted to the structure of formula (XI).

Suitable copper compounds are those already exemplified in relation to Processes D and E and the amount of copper compound is preferably from 1 to 10 parts by weight per part by weight of the conjugated polymer.

The reaction between the conjugated polymer and the amine is preferably carried out by dissolving the two reagents in a solvent and heating them, with stirring, in the presence of the copper compound. Although it is not critical to the process, we prefer that the reaction should take place under an atmosphere of an inert gas, such as nitrogen. Suitable solvents for this process are those hitherto exemplified in relation to Process A. The reaction temperature is preferably from 60°C to 200°C. The reaction time cannot be specified simply, since it depends very much on the amount of amine used and on the kind of conjugated polymer, but generally the reaction will take from 30 minutes to 48 hours.

After completion of the reaction, the reaction mixture is preferably concentrated under reduced pressure, treated with an acidic aqueous alcohol or similar reagent to remove the copper compound and filtered. The powder collected by filtration is

then preferably treated with a basic solution (such as methanolic sodium hydroxide) and filtered to give the modified conjugated polymer, generally as a black powder.

Process H

In this process, the conjugated polymer having a recurring unit of formula (I) is reacted with hydrogen sulphide, thus converting some or all of the diethynylene structures in the conjugated polymer into structures of formula (XII):

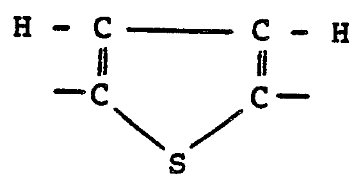

(XII)

The way in which the conjugated polymer is reacted with hydrogen sulphide is not critical to this process; however, we would normally prefer to dissolve the conjugated polymer in a suitable solvent and then effect the reaction by blowing dry hydrogen sulphide through the solution. The reaction time cannot be estimated simply, because it depends upon the rate at which the hydrogen sulphide is blown through the solution and on the nature of the conjugated polymer; however, generally the reaction will take from 30 minutes to 24 hours. The reaction temperature is preferably within the range from 20°C to 200°C. Suitable solvents are as exemplified in relation to Process A.

After completion of the reaction, we prefer that the reaction mixture should be concentrated and the concentrate mixed with a non-solvent for the conjugated polymer (such as methanol) to reprecipitate the polymer, giving the modified conjugated polymer in the form of a powder.

The various conjugated polymers produced by the processes of the invention are superior to poly(diethynylbenzene) in heat resistance; thus, whereas poly(diethynylbenzene) thermally decomposes at a temperature of 200°C or even lower, the polymers of the present invention are normally stable even at 300°C, without thermal decomposition.

Furthermore, although the aforementioned polymers having a repeating unit of formula:

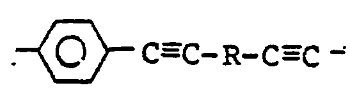

are insoluble in organic solvents, the various conjugated polymers of the present invention are soluble in a wide range of organic solvents, including chloroform, toluene, tetrahydrofuran, N,N-dimethylformamide, N-methylpyrrolidone, pyridine, aniline, hexamethylphosphoric triamide and dimethyl sulphoxide. Accordingly, it is possible to mould the conjugated polymers of the invention easily simply by casting a solution of them in a suitable solvent. Moreover, it is possible to convert the conjugated polymers of the present invention into organic semiconductors and organic

conductors having various electric conductivities by doping them to varying extents with a variety of dopants, including electron-donative compounds and electron-attractive compounds. For example, by doping the polymers of the invention with sulphuric acid, it is possible to increase electrical conductivity by as much as $10^8$ times or even more.

Thus, the conjugated polymers of the invention have excellent stability, valuable mouldability and are useful as organic semiconductors or organic conductors.

The invention is further illustrated by reference to the following Examples.

In the Examples, the degrees of polymerisation (n) of the conjugated polymers have been calculated from the number average molecular weight ($\bar{M}n'$), which, in turn, has been calculated from the polystyrene-reduced number average molecular weight ($\bar{M}n$) of the conjugated polymer, as measured in N-methylpyrrolidone as solvent, using a high temperature-high speed gel permeation chromatograph (model 150 C GPC/ALC, manufactured by Waters Co., U.S.A.) and the following Q factor (a molecular weight correction factor).

$$Q_{CP}/Q_{PS} = 0.25$$

($Q_{CP}$ is the Q factor of the conjugated polymer and $Q_{PS}$ is the Q factor of polystyrene), where the structure

of the conjugated polymer and the bulkiness of the conjugated polymer in solution are taken into consideration.

## EXAMPLE 1

### Preparation of 2,6-diethynylpyridine

This method is based upon that described in "Synthesis",page 627 (1980).

Under a stream of nitrogen, 24.8 grams (104.7 mmoles) of 2,6-dibromopyridine, 22.6 grams (230.3 mmoles) of trimethylsilylacetylene and 250 ml of diethylamine were charged into a 500 ml three-necked flask and thoroughly stirred. 1.6 grams (2.3 mmoles) of dichlorobis(triphenylphosphine)palladium and 0.44 grams (2.3 mmoles) of cuprous iodide were then added, and the mixture was stirred at room temperature for 3 hours. After completion of the reaction, the insoluble diethylammonium bromide which precipitated was removed by filtration, the filtrate was evaporated to dryness under reduced pressure and the residue was extracted with 100 ml of benzene.

The benzene solution thus obtained was evaporated to dryness under reduced pressure, giving 19.5 g of a brown solid containing 2,6-bis(trimethylsilylethynyl)-pyridine. The whole of this solid was charged, under a stream of nitrogen, together with 100 ml of methanol into a 300 ml three-necked flask. 145 ml of a 1 N aqueous solution of potassium hydroxide was then dropped into the flask, and the mixture was stirred at room temperature for 1 hour. The methanol was distilled off under reduced pressure, the residue was extracted with diethyl ether, and the diethyl ether was then distilled under reduced pressure from the extract, giving a brown solid. This solid was sublimed at a pressure of 1 Torr and at 40°C, to give 6.6 g (52 millimoles) of 2,6-diethynylpyridine in the form of needle-like crystals.

(b) Preparation of poly(2,6-diethynylpyridine)

0.072 g (0.73 millimole) of cuprous chloride, 0.085 g (0.73 millimole) of N,N,N',N'-tetramethylethylenediamine, 300 ml of nitrobenzene and 1 ml of pyridine were placed in a 500 ml three-necked flask equipped with a reflux condenser and with a tube for blowing oxygen through the contents. The mixture was then stirred. To the resulting solution were added 2.47 g

(19.4 millimoles) of the 2,6-diethynylpyridine obtained as described above. Whilst maintaining the reaction temperature at 100-110°C and continuing stirring, oxygen was blown through the solution for 2 hours. At the end of this time, the solution was concentrated by evaporation under reduced pressure, the concentrate was added to 500 ml of methanol containing 5 ml of 37% hydrochloric acid, and the resulting mixture was stirred and filtered. The powder thus obtained was added to a 2% w/w methanolic solution of sodium hydroxide, and the resulting mixture was stirred for 2 hours. The mixture was then filtered, giving 2.3 g of a black powder.

Measurement of the infrared absorption spectrum revealed that, although absorption due to the ethynyl group at about 3 300 $cm^{-1}$ (which is observable in 2,6-diethynylpyridine) had disappeared, absorption still existed at about 2 200 $cm^{-1}$ (due to the carbon-carbon triple bond) and there was also absorption at about 1 550 $cm^{-1}$ (due to the pyridine ring). On the basis of this, the product was determined to have the structure of poly(2,6-diethynylpyridine).

The characteristic infrared absorption spectrum (Nujol-trade mark-mull) of the product was $\nu cm^{-1}$:

3050, 2220, 2150, 1563, 1547, 1200, 807, 723.

The number average molecular weight ($\bar{M}n'$) of the product was 14,000, the degree of polymerisation (n) was 112 and its melting point (decomposition point) was at least 300°C.

Example 2

(a) 2,5-diethynylthiophene

Under a stream of nitrogen, 11.8 g (48.8 millimoles) of 2,5-dibromothiophene, 11.4 g (116 millimoles) of trimethylsilylacetylene and 200 ml of diethylamine were placed in a 500 ml three-necked flask, and the mixture was thoroughly stirred. 1.1 g (1.6 millimoles) of dichlorobis(triphenylphosphine)palladium and 0.3 g (1.6 millimoles) of cuprous iodide were then added, and the mixture was heated, with stirring, at 60°C for 4 hours. The mixture was then cooled, the insoluble diethylammonium bromide was removed by filtration, and the filtrate was evaporated to dryness under reduced pressure. The residue was then extracted with 50 ml of benzene and the extract was evaporated to dryness under reduced pressure. The resulting residue was refluxed, with stirring, in 100 ml of acetone, together with 5 g of activated carbon. The activated carbon was then filtered off and the acetone was removed, giving 11.5 g (41.5 millimoles) of 2,5-bis(trimethylsilylethynyl)thiophene, in the form of white crystals.

The whole of this 2,5-bis(trimethylsilylethynyl)thiophene was then charged, with 70 ml of methanol, into a 500 ml three-necked flask, under a stream of nitrogen. 70 ml of a saturated methanolic solution of potassium fluoride were then dropped into the mixture, which was then heated, with stirring, at 40°C for 3 hours. At the end of this time, the methanol was distilled off under reduced pressure and the residue was extracted with diethyl ether. The residual potassium fluoride was separated from the extracted solution, and then the diethyl ether was removed by evaporation under reduced pressure, to give 4.6 g of 2,5-diethynylthiophene, in the form of a yellow liquid.

(b) Preparation of poly(2,5-diethynylthiophene)

The procedure of step (b) of Example 1 was repeated, but using 2.5 g of (19.4 millimoles) of the 2,5-diethynylthiophene obtained above, to give 2.3 g of a black powder.

Measurement of the infrared spectrum revealed that the absorption peak at about 3 300 $cm^{-1}$ due to the ethynyl group, which is observable in 2,5-diethynylthiophene, had disappeared; however, the absorption due to the carbon-carbon triple bond at about 2 200 $cm^{-1}$ and the absorption due to the thiophene ring at about 800 $cm^{-1}$ still appeared. As a result, this product was determined to be poly(2,5-diethynylthiophene).

The characteristic infrared absorption spectrum (Nujol mull) of this product was, $\nu\ cm^{-1}$:

2180, 2140, 800, 720.

$\bar{M}n'$ was 20,000 and n was 154.

Example 3

(a) Preparation of 2,5-diethynylthiophene

Under a stream of nitrogen, 25 g (103 millimoles) of 2,5-dibromothiophene, 10.4 g (124 millimoles) of 2-methyl-3-butyn-2-ol and 250 ml of diethylamine were placed into a 500 ml three-necked flask equipped with a reflux condenser. The mixture was stirred, and then 1.45 g (2.1 millimoles) of dichlorobis(triphenylphosphine)palladium and 0.20 g (1.0 millimole) of cuprous iodide were added thereto. The mixture was then stirred at room temperature for 15 hours, after which the insoluble diethylammonium bromide was removed by filtration. The filtrate was then concentrated by evaporation under reduced pressure and the residue was extracted with diethyl ether. The ethereal solution was concentrated under reduced pressure, and petroleum ether was added to the residual oily product, to solidify it, giving 20.6 g (yield 80%) of a light brown powder, melting at 118-120°C.

Nuclear Magnetic Resonance Spectrum (deuteroacetone) δ ppm:

1.49 (12H, singlet, 4 x $CH_3$);

4.40 (2H, singlet, 2 x OH);

7.03 (2H, singlet, 2 hydrogens of thiophene).

Moreover, the infrared absorption spectrum (Nujol mull) showed the following peaks, ν $cm^{-1}$:

3310 (OH);

2220 (carbon-carbon triple bond);

810 (thiophene ring).

On the basis of this, the product was considered to be 2,5-bis(3-hydroxy-3-methyl-1-butynyl)thiophene, which has the following formula:

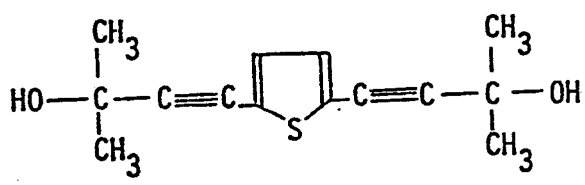

5.0 g (20.2 millimoles) of this 2,5-bis(3-hydroxy-3-methyl-1-butynyl)thiophene and 120 ml of toluene were then charged, under a stream of nitrogen, into a 300 ml three-necked flask. 0.80 g (14.3 millimoles) of powdered potassium hydroxide were then added to the mixture, and nitrogen was blown into the mixture at a temperature of 65°C for 3 hours, whilst removing the ketone, which formed as a by-product. After completion of the reaction,

the solids were removed by filtration and the toluene in the filtrate was distilled off under reduced pressure. The resulting residue was distilled under reduced pressure, to give 2.45 g (yield 92%) of a light yellow oily product, boiling at 40°C/0.1 mm Hg.

Nuclear Magnetic Resonance Spectrum ($CDCl_3$) $\delta$ ppm:

3.37 (2H, singlet, 2 x ethynyl);

7.23 (2H, singlet, hydrogens of thiophene).

Infrared Absorption Spectrum (neat) $\nu$ $cm^{-1}$:

3280 (ethynyl);

2100 (carbon-carbon triple bond);

800 (thiophene ring).

On the basis of these observations, the product was determined to be 2,5-diethynylthiophene.

(b) Preparation of 2,5-diethynylthiophene copolymer

2.25 g (17.1 millimoles) of 2,5-diethynylthiophene, 4.04 g (17.1 millimoles) of 2,6-dibromopyridine, 50 ml of diethylamine and 150 ml of *N*-methylpyrrolidone were placed in a 300 ml three-necked eggplant-type flask, and thoroughly stirred. 0.24 g (0.34 millimole)

of dichlorobis(triphenylphosphine)palladium and 0.033 g (0.17 millimole) of cuprous iodide were added to the solution in the flask and the mixture was heated, with stirring, at 60°C for 2 hours. At the end of this time, the solution was concentrated by evaporation under reduced pressure, the resulting solid was washed with 300 ml of water containing 5 ml of 37% hydrochloric acid and finally it was washed with 300 ml of 2% w/w methanolic sodium hydroxide, to give 3.0 g of a dark brown powder.

In the infrared absorption spectrum (Nujol mull) of this powder, the absorption at about 3 300 $cm^{-1}$ due to the C-H stretching vibration of the ethynyl group, which is observed in 2,5-diethynylthiophene, had disappeared, whilst the absorption at about 2 100 $cm^{-1}$ due to the carbon-carbon triple bond in 2,5-diethynylthiophene had shifted by about 100 $cm^{-1}$ towards a higher wave number. Furthermore, there appeared a new absorption at about 1 560 $cm^{-1}$, which is characteristic of the pyridine ring. On the basis of these observations, the product was considered to be a polymer having the following structure, in which an ethynylene group and a thiophene or pyridine ring are linked alternately; this may be represented by the formula:

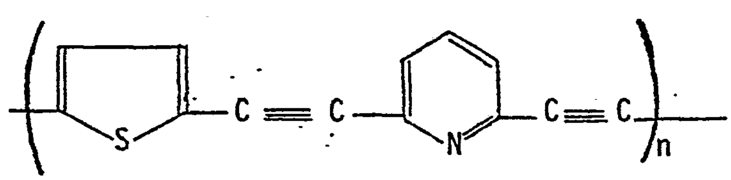

The infrared absorption spectrum of this polymer (Nujol mull) was, ν $cm^{-1}$:

3050, 2200, 1570, 1550, 800, 720, 660.

Mn' was 20,000, n was 97 and the melting point (decomposition point) was 300°C or higher.

Example 4

The procedure of Example 3(b) was repeated, except that the 2,6-dibromopyridine was replaced by 4.14 g (17.1 millimoles) of 2,5-dibromothiophene, to give 3.25 g of a dark brown powder.

Measurement of the infrared absorption spectrum revealed that the absorption at about 3 300 $cm^{-1}$, which is due to the ethynyl group in 2,5-diethynylthiophene, had disappeared, whilst the absorption due to the carbon-carbon triple bond had shifted by 60 $cm^{-1}$ towards a higer wave number, so that it was observed at 2 160 $cm^{-1}$. Furthermore, the characteristic absorption at 798 $cm^{-1}$ of the thiophene ring was present. On the basis of this, the product was determined to have a structure which could be represented by the following formula:

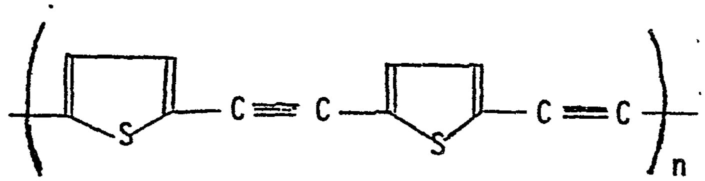

Infrared absorption spectrum (Nujol mull) $\nu$ $cm^{-1}$: 2160, 798, 720, 660.

$\bar{M}n'$ was 11 000, $\bar{n}$ was 52 and the melting point (decomposition point) was 300°C or higher.

Example 5

(a) Preparation of 2,6-diethynylpyridine

Under a nitrogen atmosphere, 25 g (106 millimoles) of 2,6-dibromopyridine, 21.3 g (253 millimoles) of 2-methyl-3-butyn-2-ol and 300 ml of diethylamine were placed in a 500 ml three-necked flask equipped with a reflux condenser. After stirring the mixture, 1.48 g (2.1 millimoles) of dichlorobis(triphenylphosphine)palladium and 0.21 g (1.1 millimoles) of cuprous iodide were added and the mixture was allowed to react, with sufficient stirring, at room temperature for 15 hours. At the end of this time, the insoluble diethylammonium bromide was removed by filtration, the filtrate was concentrated by evaporation under reduced pressure, and the residue was extracted with diethyl ether. The ether was distilled off under reduced pressure from the ethereal extract and the remaining oily product was purified by sublimation at 105°C and at a pressure of 0.5 mm Hg, to give 16.7 g (yield 65%) of a white crystalline product, melting at 112-114°C.

The nuclear magnetic resonance spectrum (in deuteroacetone, $\delta$ ppm) revealed the existence of methyl protons (1.56, singlet, 12H), hydroxy protons (4.71, singlet, 2H) and protons at the 3-, 4- and 5- positions of the pyridine ring (7.43, multiplet, 3H). Furthermore, the infrared absorption spectrum (Nujol mull) revealed the existence of a hydroxy group (3370 $cm^{-1}$), a carbon-carbon triple bond (2220 $cm^{-1}$) and a pyridine ring (1540, 1560, 790 $cm^{-1}$). On the basis of these observations, the product was determined to be 2,6-bis(3-hydroxy-3-methyl-1-butynyl)pyridine.

5.0 g (20.6 millimoles) of this 2,6-bis(3-hydroxy-3-methyl-1-butynyl)pyridine and 150 ml of toluene were charged, under an atmosphere of nitrogen, into a 300 ml three-necked flask. 0.81 g (14.4 millimoles) of powdered potassium hydroxide was added, and the mixture was stirred at 80°C for 2 hours whilst removing the acetone formed by the reaction by blowing nitrogen gas through the reaction system. After completion of the reaction, the solid was removed by filtration and the toluene in the filtrate was removed by distillation under reduced pressure. The resulting residue was purified by sublimation at 60°C and at a pressure of 0.5 mm Hg, to give 1.83 g (yield 70%) of colourless needle-like crystals melting at 71°C.

The nuclear magnetic resonance spectrum (in $CDCl_3$, $\delta$ ppm) revealed the existence of ethynyl protons (3.15, singlet, 2H),

and protons at the 3-, 4- and 5- positions of a pyridine ring (7.70, multiplet, 3H). Moreover, the infrared absorption spectrum (Nujol mull) revealed the existence of an ethynyl group (3270 $cm^{-1}$), a carbon-carbon triple bond (2100, 2020 $cm^{-1}$) and a pyridine ring (1550, 1570, 800 $cm^{-1}$). On the basis of this, the product was determined to be 2,6-diethynylpyridine.

(b) Preparation of 2,6-diethynylpyridine copolymer

The procedure described in Example 3(b) was repeated, except that 2.50 g (19.7 millimoles) of the 2,6-diethynylpyridine obtained above and 4.67 g (19.7 millimoles) of 2,6-dibromopyridine were used, to give 3.60 g of a dark brown powder.

The infrared absorption spectrum revealed that the absorption at about 3300 $cm^{-1}$, observable in 2,6-diethynylpyridine and due to the ethynyl groups, had disappeared, that the absorption due to the carbon-carbon triple bond had shifted by 80 $cm^{-1}$ towards a higher wave number so that it was observed at about 2180 $cm^{-1}$, and that the characteristic absorption of the pyridine ring existed at about 1550 $cm^{-1}$. On the basis of these observations, the product was determined to have a structure which could be represented by the following formula:

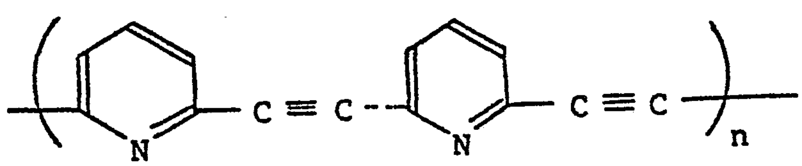

The characteristic absorptions in the infrared absorption spectrum of this conjugated polymer were, $\nu$ $cm^{-1}$:

3050, 2180, 1565, 1545, 1200, 805, 720.

$\bar{M}n'$ was 12 000, n was 59 and the melting point (decomposition point) was 300°C or higher.

Example 6

30 ml of a solution containing 2.64 g (20 millimoles) of 2,5-diethynylthiophene in tetrahydrofuran were dropped into 50 ml of a solution of ethylmagnesium bromide [prepared from 1 g (41 millimoles) of magnesium and 4.4 g (41 millimoles) of ethyl bromide] in tetrahydrofuran. The resulting mixture was stirred at room temperature for 1 hour. To the resulting Grignard reagent solution of 2,5-diethynylthiophene thus obtained were added 4.7 g (20 millimoles) of 2,6-dibromopyridine, 0.045 g (0.2 millimole) of palladium acetate and 0.11 g (0.4 millimole) of triphenylphosphine, the palladium acetate and triphenylphosphine being catalysts. The resulting mixture was then refluxed for 3 hours. At the end of this time, the resulting solid was separated and washed with 300 ml of water containing 5 ml of 37% hydrochloric acid, and then with 300 ml of a 2% w/w methanolic solution of sodium hydroxide, to give 3.2 g of a dark brown powder.

The infrared absorption spectrum of this powder was measured and, in the same way as was done in Example 1, the product was found to be a polymer having a structure which can be represented by the following formula:

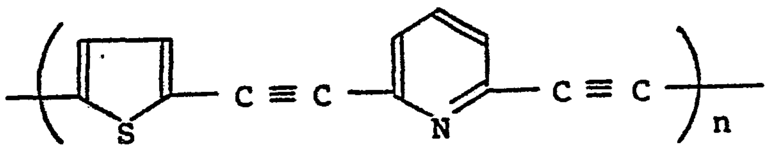

$\overline{M}n'$ was 14 000, n was 68 and the melting point (decomposition point) was 300°C or higher.

Example 7

Under a stream of nitrogen, 0.5 g of poly(2,6-diethynylpyridine), obtained as described in Example 1, 300 ml of N-methylpyrrolidone, 60 ml of hexamethylphosphoric triamide, 2 g of cuprous chloride and 7.3 g (78 millimoles) of aniline were placed in a 500 ml three-necked flask and allowed to react at 150°C for 2 hours. After concentrating the reaction mixture, the concentrate was added to 500 ml of methanol, which had been acidified with hydrochloric acid, and the mixture was stirred and filtered, giving 0.3 g of a powder.

Measurement of the infrared absorption spectrum of this powder revealed that the absorption due to the carbon-carbon

triple bond [observable in poly(2,6-diethynylpyridine)] at about 2200 $cm^{-1}$ had disappeared whilst a new absorption peak attributable to a pyrrole ring had appeared at about 835 $cm^{-1}$, and the absorption due to the pyridine ring still remained. On the basis of these observations, the product was considered to have the following structure:

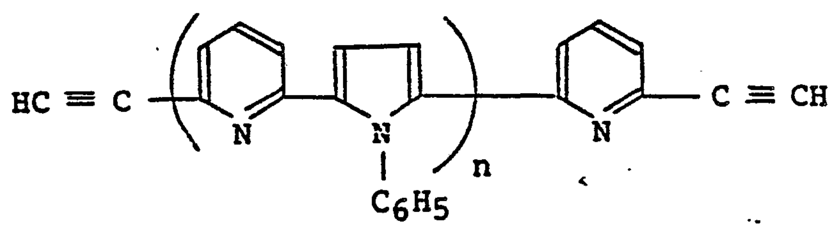

Infrared absorption spectrum (Nujol mull), $\nu cm^{-1}$: 1580, 1560, 1545, 1200, 835, 807, 720.

$\bar{M}n'$ was 31000, n was 142 and the melting point (decomposition point) was 300°C or higher.

EXAMPLE 8

The procedure described in Example 7 was repeated, except that 1 g of poly(2,5-diethynylthiophene), prepared as described in Example 2, was used in place of the poly(2,6-diethynylpyridine), giving 0.5g of a powder.

Measurement of the infrared absorption spectrum of this powder revealed that the absorption ascribed to the carbon-carbon triple bond at about 2200 $cm^{-1}$ had disappeared, a new absorption ascribable to a pyrrole ring had appeared at 835 $cm^{-1}$, and the absorption ascribable to the thiophene ring remained. On the basis of these observations, the product was considered to have the following structure:

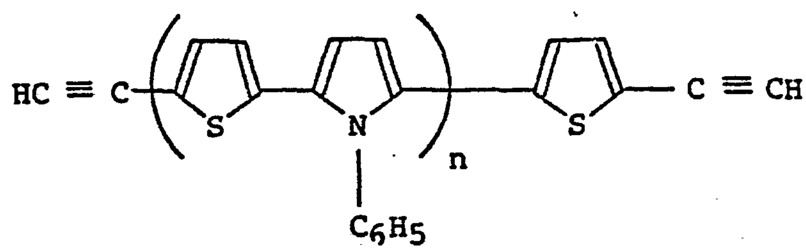

Infrared absorption spectrum (Nujol mull), $\nu cm^{-1}$: 1580, 835, 800, 720.

$\bar{M}n'$ was 30000, n was 134 and the melting point (decomposition point) was 300°C or higher.

EXAMPLE 9

Following the procedure described in Example 7, 1 g of poly(2,6-diethynylpyridine), 600 ml of N-methylpyrrolidone, 120 ml of hexamethylphosphoric triamide, 4 g of cuprous chloride and 0.4 g (4.3 mmoles) of aniline were reacted at 150°C for 1 hour and then subsequently treated as in Example 7, to give 0.6 g of a powder.

Measurement of the infrared absorption spectrum of this powder revealed that the absorptions at about 2200 $cm^{-1}$ and 1550 $cm^{-1}$ ascribed to the carbon-carbon triple bond and the pyridine ring, respectively, remained whilst a new absorption ascribable to a pyrrole ring had appeared at 835 $cm^{-1}$. On the basis of these observations, the product was considered to have the structure of poly(2,6-diethynylpyridine) in which some of the diethynylene units had been replaced by units of the following structure:

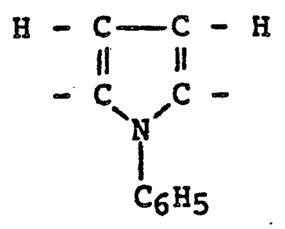

Infrared absorption spectrum (Nujol mull), $\nu cm^{-1}$: 3050, 2220, 2150, 1580, 1560, 1545, 1200, 835, 807, 720.

$\bar{M}n'$ was 20000 and the melting point was 300°C or higher.

## EXAMPLE 10

Following the procedure described in Example 7, 1 g of poly(2,5-diethynylthiophene), 600 ml of N-methylpyrrolidone, 120 ml of hexamethylphosphoric triamide, 4 g of cuprous chloride and 0.35 g (3.8 mmoles) of aniline were reacted at 150°C for 1 hour and then treated as in Example 7, to give 0.5 g of a powder.

Measurement of the infrared absorption spectrum of this powder revealed that the absorption at about 2200 $cm^{-1}$ and 800 $cm^{-1}$ ascribed to the carbon-carbon triple bond and the thiophene ring, respectively, remained, whilst a new absorption ascribable to a pyrrole ring had appeared at 835 $cm^{-1}$. On the basis of these observations, the product was considered to have the structure of poly(2,5-diethynylthiophene) in which some of the diethynylene structural units had been replaced by units of the following structure:

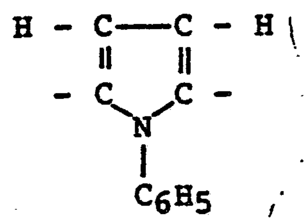

Infrared absorption spectrum (Nujol mull) $\nu$ $cm^{-1}$:
2180, 2140, 1580, 835, 800, 720.

$\bar{M}n'$ was 23000 and the melting point (decomposition point) was 300°C or higher.

EXAMPLE 11

1 G of poly(2,5-diethynylthiophene), prepared as described in Example 2, 300 ml of N-methylpyrrolidone and 60 ml of hexamethylphosphoric triamide were placed in a 500 ml three-necked flask and caused to dissolve by heating to 100°C. The solution was then cooled to 60°C, after which dry hydrogen sulphide gas was blown through it, with stirring, for 6 hours. The reaction mixture was then concentrated by evaporation under reduced pressure and the concentrate was dropped into 300 ml of methanol, to give 0.6 g of a black powder.

Measurement of the infrared absorption spectrum of the product revealed that the absorption at about 2200 $cm^{-1}$ ascribed to the carbon-carbon triple bond had disappeared, whilst an intense absorption ascribable to a thiophene ring had appeared at 795 $cm^{-1}$.

On the basis of these observations, the product was considered to have the following structure:

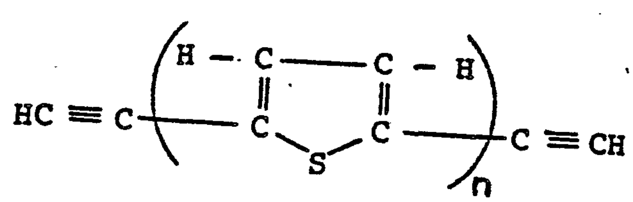

Infrared absorption spectrum (Nujol mull), $\nu cm^{-1}$:
795, 695.

$\bar{M}n'$ was 23000 and the melting point (decomposition point) was 300°C or higher.

EXAMPLE 12

Following the procedure described in Example 11, dry hydrogen sulphide was blown for 1 hour into a solution containing 1 g of poly(2,5-diethynylthiophene) at 60°C, and then the product was treated as in Example 11, to give 0.65 g of a black powder.

Measurement of the infrared absorption spectrum of this powder revealed that the intensity of absorption at about 2200 $cm^{-1}$ ascribed to the carbon-carbon triple bond was weaker than that in the starting material and that an intense absorption ascribable to the thiophene ring had appeared at 795 $cm^{-1}$. On the basis of these observations, the product was considered to have the structure of poly(2,5-diethynylthiophene) in which some of the diethynylene units had been replaced by units of the following structure:

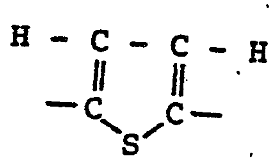

Infrared absorption spectrum (Nujol mull), $\nu cm^{-1}$:
2180, 2140, 795, 720, 697.

Mn' was 21000 and the melting point (decomposition point) was 300°C or higher.

Example 13

0.5 grams of poly(2,6-diethynylpyridine), obtained as described in Example 1, 300 ml of N-methylpyrrolidone and 60 ml of hexamethylphosphoric triamide were placed in a 500 ml three-necked flask and caused to dissolve by heating to 100°C. The resulting solution was then cooled to 60°C and dry hydrogen sulphide gas was blown through it, with stirring, for 6 hours. The solution was then concentrated by evaporation under reduced pressure and the concentrate was dropped into 300 ml of methanol to give 0.25 g of a black powder.

Measurement of the infrared absorption spectrum of this powder revealed that the absorption at about 2200 $cm^{-1}$ ascribed to the carbon-carbon triple bond had disappeared, a new absorption ascribed to the thiophene ring had appeared at 800 $cm^{-1}$, and the absorption at about 1550 $cm^{-1}$ ascribed to the pyridine ring remained. On the basis of these observations, the product was considered to have the following structure.

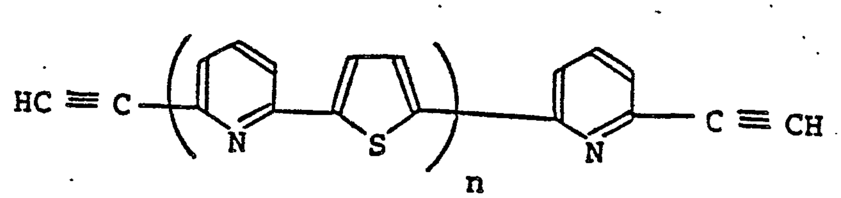

Infrared absorption spectrum (Nujol mull), $\nu cm^{-1}$:
1561, 1545, 1200, 807, 800, 720.

Mn' was 23000 and the melting point (decomposition point) was 300°C or higher.

EXAMPLE 14

Following the procedure described in Example 13, dry hydrogen sulphide gas was blown for 1 hour through a solution containing 0.5 grams of poly(2,6-diethynylpyridine) at 60°C, and then the product was treated as described in Example 13, to give 0.24 grams of a black powder.

Measurement of the infrared absorption spectrum of this powder revealed that the absorptions at about 2200 $cm^{-1}$ and 1550 $cm^{-1}$, ascribed, respectively, to the carbon-carbon triple bond and the pyridine ring remained, whilst a new absorption ascribable to the thiophene ring had appeared at 800 $cm^{-1}$. On the basis of these observations, the product was considered to have the structure of poly(2,6-diethynylpyridine) in which some of the diethynylene units had been replaced by units of the following structure:

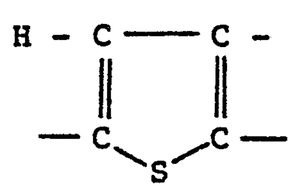

Infrared absorption spectrum (Nujol mull) $\nu cm^{-1}$:
2220, 2150, 1560, 1545, 1200, 807, 800, 720.

$\bar{M}n'$ was 17000 and the melting point (decomposition point) was 300°C or higher.

CLAIMS:-

1. A conjugated polymer having a recurring unit of formula (I) or (II):

$$-C\equiv C-Ar-C\equiv C- \qquad (I)$$

$$-Ar-C\equiv C-Ar'-C\equiv C- \qquad (II)$$

in which Ar and Ar', which may be the same or different, each represent heteroaromatic rings.

2. A polymer according to claim 1, having a recurring unit of formula (I) in which Ar represents a thiophene ring, a pyridine ring, a furan ring or a pyrrole ring.

3. A conjugated polymer according to claim 1, having a recurring unit of formula (II) in which Ar and Ar' are the same or different and each represents a thiophene ring, a pyridine ring, a furan ring or a pyrrole ring.

4. A process for producing a conjugated polymer having a recurring unit of formula (I), as claimed in claim 1 or claim 2, which comprises subjecting a diethynyl-heteroaromatic compound of formula (III):

$$HC\equiv C\text{-}Ar\text{-}C\equiv CH \qquad (III)$$

(wherein Ar is as defined in claim 1 or claim 2) to an oxidative coupling reaction with oxygen in the presence of a catalyst comprising a copper compound and an amine compound.

5. A process according to claim 4, in which said diethynyl-heteroaromatic compound is prepared by reacting a dihalo-heteroaromatic compound with an ethynylsilyl compound of formula (VI):

$$R^1R^2R^3SiC\equiv CH \qquad (VI)$$

(in which $R^1$, $R^2$ and $R^3$ are the same or different and each represents an alkyl, aryl or alkenyl group) in the presence of a dehalogenating agent and a catalyst comprising a palladium compound and a copper compound, and then desilylating the reaction product with a metal halide.

6. A process according to claim 4, in which said diethynyl-heteroaromatic compound is prepared by reacting a dihalo-heteroaromatic compound with a propargyl alcohol derivative in the presence of a de-halogenating agent and a catalyst comprising a palladium compound and a copper compound, and then reacting the reaction product with a de-ketonating agent to remove the ketone group.

7. A process for preparing a conjugated polymer having a recurring unit of formula (II), as claimed in claim 1 or claim 3, in which a dihalo-heteroaromatic compound of formula (IV):

$$X-Ar'-X \qquad (IV)$$

(wherein Ar' is as defined in claim 1 or claim 3 and X represents a halogen atom) is reacted with a diethynyl-heteroaromatic compound of formula (III):

$$HC{\equiv}C-Ar-C{\equiv}CH \qquad (III)$$

(wherein Ar is as defined in claim 1 or claim 3) in the presence of an amine compound and of a catalyst comprising a palladium compound and a copper compound.

8. A process according to claim 7, in which said diethynyl-heteroaromatic compound is prepared by reacting a dihalo-heteroaromatic compound with an ethynylsilyl compound of formula (VI):

$$R^1R^2R^3SiC{\equiv}CH \qquad (VI)$$

(in which $R^1$, $R^2$ and $R^3$ are the same or different and each represents an alkyl, aryl or alkenyl group) in the presence of a dehalogenating agent and a catalyst comprising a palladium compound and a copper compound, and then desilylating the reaction product with a metal halide.

9. A process according to claim 7, in which said diethynyl-heteroaromatic compound is prepared by reacting a dihalo-heteroaromatic compound with a propargyl alcohol derivative in the presence of a de-halogenating agent and a catalyst comprising a palladium compound and a copper compound, and then reacting the reaction product with a de-ketonating agent to remove the ketone group.

10. A process for preparing a conjugated polymer having a recurring unit of formula (II), as claimed in claim 1 or claim 3, in which a dihalo-heteroaromatic compound of formula (IV):

$$X\text{-}Ar'\text{-}X \qquad (IV)$$

(in which Ar' is as defined in claim 1 or claim 3 and X represents a halogen atom) is reacted with a di-Grignard reagent of formula (V):

$$X'MgC{\equiv}C\text{-}Ar\text{-}C{\equiv}CMgX' \qquad (V)$$

(in which Ar is as defined in claim 1 or claim 3 and X' represents a halogen atom, which may be the same as or different from that represented by X) in the presence of a palladium- and phosphorus-containing catalyst.

11. A process as claimed in claim 10, in which said catalyst comprises a palladium compound and a phosphorus compound.

12. A process as claimed in claim 10, in which said catalyst comprises a compound containing a palladium and a phosphorus atom.

13. A process for modifying a conjugated polymer having a recurring unit of formula (I), as claimed in claim 1 or claim 2, in which said conjugated polymer is reacted with an amine compound of formula $NH_2R^7$ (in which $R^7$ represents a hydrogen atom or an alkyl, aryl or aralkyl group), in the presence of a copper compound.

14. A process as claimed in claim 13, in which said amine compound is employed in an amount of from 5 to 100 equivalents per diethynylene unit in said polymer.

15. A process for modifying a conjugated polymer having a recurring unit of formula (I), as claimed in claim 1 or claim 2, which comprises reacting said polymer with hydrogen sulphide.